# EUROPEAN PATENT SPECIFICATION

(11) **EP 1 845 951 B1**
(45) Date of publication and mention of the grant of the patent: **15.09.2010**
(21) Application number: 06719816.8
(22) Date of filing: 27.01.2006
(51) Int. Cl.: A61K 9/16, A61P 7/00, A61P 9/00

(54) **EMBOLIZATION USING POLY-4-HYDROXYBUTYRATE PARTICLES**
EMBOLISIERUNG UNTER VERWENDUNG VON POLY-4-HYDROXYBUTYRAT-PARTIKELN
EMBOLISATION UTILISANT DES PARTICULES DE POLY-4-HYDROXYBUTYRATE

(30) Priority: 28.01.2005 US 648052 P
(43) Date of publication of application: 24.10.2007
(73) Proprietor: Tepha, Inc., Lexington MA 02421 (US)
(72) Inventor: MARTIN, David, P., Arlington, Massachusetts 02474 (US); CRABTREE, Donald, Boston, Massachusetts 02116 (US); WILLIAMS, Simon, F., Sherborn, Massachusetts 01770 (US)
(74) Representative: Wright, Andrew John
(86) International application number: PCT/US2006/003128
(87) International publication number: WO 2006/081517

(56) References cited:
- US-A1- 2005 267 516
- US-A1- 2005 267 516
- PATENT ABSTRACTS OF JAPAN vol. 017, no. 627 (C-1131), 19 November 1993 (1993-11-19) & JP 05 194141 A (MITSUBISHI KASEI CORP), 3 August 1993 (1993-08-03)
- KASSAB A CH ET AL: "Embolization with polyhydroxybutyrate (PHB) microspheres: In-vivo studies" JOURNAL OF BIOACTIVE AND COMPATIBLE POLYMERS, vol. 14, no. 4, July 1999 (1999-07), pages 291-292, XP009066540 ISSN: 0883-9115
- KASSAB ET AL: "Rifampicin carrying polyhydroxybutyrate microspheres as a potentail chemoembolization agent" JOURNAL OF BIOMATERIALS SCIENCE. POLYMER EDITION, vol. 8, no. 12, 1 January 1997 (1997-01-01), pages 947-961, XP009093808
- ZHANG ET AL: "Biodegradable Polymer Blends of Poly(3-hydroxybutyrate and Poly(DL-lactide)-co-poly(ethylene glycol)" JOURNAL OF APPLIED POLYMER SCIENCE, vol. 65, no. 10, - 1997 pages 1849-1856,
- KASSAB ET AL: "Rifampicin carrying polyhydroxybutyrate microspheres as a potentail chemoembolization agent" JOURNAL OF BIOMATERIALS SCIENCE. POLYMER EDITION, vol. 8, no. 12, 1 January 1997 (1997-01-01), pages 947-961, XP009093808
- ZHANG ET AL: "Biodegradable Polymer Blends of Poly(3-hydroxybutyrate and Poly(DL-lactide)-co-poly(ethylene glycol)" JOURNAL OF APPLIED POLYMER SCIENCE, vol. 65, no. 10, - 1997 pages 1849-1856,

## Description

### FIELD OF THE INVENTION

The present invention generally relates to the use of poly-4-hydroxybutyrate and its copolymers in embolization, methods for using these materials in embolization, and processes for producing such materials.

### BACKGROUND OF THE INVENTION

Embolizations (therapeutic vascular occlusions) are used to treat or prevent a range of pathological conditions *in situ,* including, for example, tumors, vascular malformations, and hemorrhagic processes. They can be performed in a variety of vessels or organs whether healthy or diseased. In these procedures, particulate occlusion agents (emboli) are positioned in the circulatory system using catheters under imagery control. U.S. Patent No. 6,680,046 to Boschetti reports the following benefits of embolization. In the case of tumors, vascular occlusion can suppress pain, limit blood loss during surgical intervention following embolization or even bring on tumoral necrosis and avoid the necessity for surgical intervention. In the case of vascular malformations, embolization enables the blood flow to the "normal" tissues to be normalized, aids in surgery and limits the risk of hemorrhage. In hemorrhagic events or processes, vascular occlusion produces a reduction of blood flow, which promotes cicatrization of the arterial opening(s). Further, depending on the pathological conditions treated, embolization can be used for temporary as well as permanent objectives.

A range of solid materials, including polyvinylalcohol and polyacrylamide, have been used in embolization procedures. Several patents have also disclosed the combination of some of these materials with imaging and active agents, such as cell adhesion promoters. For example, U.S. Patent No. 5,635,215 discloses microspheres comprising a hydrophilic acrylic copolymer coated with a cell adhesion promoter and a marking agent, which are useful for embolization. U.S. Patent No. 5,648,100 discloses an injectable solution for therapeutic embolization, comprising microspheres comprising a hydrophilic acrylic copolymer coated with a cell adhesion promoter and a marking agent, and method of use.

Particles used in embolization should preferably be uniform in shape, and of a defined size range. Notably there have been reports of serious complications when irregular particles have been used in embolization. For example, it has been reported that two infants with symptomatic hepatic arteriovenous malformation died after embolization with polyvinylalcohol particles, and that the heterogeneity of particle size very probably contributed to the death of the infants (see U.S. Patent No. 6,680,046 to Boschetti).

There is thus a need to develop particles for embolization that are uniform in shape, and have defined size. It is also desirable to develop absorbable particles for embolization that subsequently degrade so that no foreign body is left indefinitely after embolization.

It is therefore an object of this invention to provide a composition for embolization that is degradable *in vivo.*

It is another object of this invention to provide embolization particles that do not aggregate, can be combined with other components to aid delivery, and/or can incorporate drugs and other agents or actives.

It is yet another object of this invention to provide a method for prophylactic or therapeutic embolization in a human or animal.

### Summary of the Invention

Methods to produce biocompatible particles of poly-4-hydroxybutyrate or its copolymers for embolization have been developed. These particles are absorbable, unlike currently available embolization particles, and will degrade so that no foreign body is left behind indefinitely after embolization. The particles may comprise other components such as imaging agents, contrast agents, or dyes, cell adhesion factors, anti-angiogenic agents, and/or drugs (that can be eluted and used for example in chemoembolization for the treatment of cancers).

### Detailed Description of the Invention

Biocompatible particles for embolization have been developed that are absorbable.

### I. Definitions

"Biocompatible" as generally used herein means the biological response to the material or device is appropriate for the device's intended application *in vivo.* Any metabolites of these materials should also be biocompatible.

"Poly-4-hydroxybutyrate" as generally used herein means a homopolymer comprising 4-hydroxybutyrate units. It may be referred to herein as P4HB, PHA4400 or TephaFLEX™ biomaterial (manufactured by Tepha, Inc., Cambridge, MA).

"Copolymers of poly-4-hydroxybutyrate" as generally used herein means any polymer comprising 4-hydroxybutyrate with one or more different hydroxy acid units.

"Absorbable" as generally used herein means the complete degradation of the material over time.

### II. Microparticles

### Polymers

The particles may be formed from absorbable polymers, such as poly-4-hydroxybutyrate, and copolymers thereof, such as poly-4-hydroxybutyrate-co-3-hydroxybutyrate and poly-4-hydroxybutyrate-co-glycolic acid. Tepha, Inc. of Cambridge, MA produces poly-4-hydroxybutyrate and copolymers thereof using transgenic fermentation methods.

Tepha, Inc. (Cambridge, MA) produces an absorbable biocompatible biomaterial known as TephFLEX™ (poly-4-hydroxybutyrate), and related copolymers for medical use. Related copolymers include 4-hydroxybutyrate copolymerized with 3-hydroxybutyrate or glycolic acid (U.S. Patent No. 6,316,262 to Huisman et al.*,* and U.S. Patent No. 6,323,010 to Skraly et al.)*,* typically in a ratio of up to 30 wt% P4HB. Methods to control the molecular weight of these polymers are disclosed in U.S. Patent No. 5,811,272 to Snell et al*.,* and methods to purify these polymers for medical use are disclosed in U.S. Patent No. 6,245,537 to Williams et al*.* U.S. Patent No. 6,548,569 to Williams et al*.* and WO 99/32536 to Martin et al*.* disclose the degradation rates of these polymers *in vivo* as well as their use as tissue engineering scaffolds. Other applications of these polymers have been reviewed in Williams, S.F., et al. Applications of PHAs in Medicine and Pharmacy, in Biopolymers, Polyesters, III Vol. 4:91-127 (2002).

Poly-4-hydroxybutyrate belongs to a larger class of materials called polyhydroxyalkanoates, and is usually produced by transgenic fermentation. The polymer cannot be readily synthesized by chemical means with sufficiently high molecular weight for most applications. It is distinguished by its physical and thermal properties, and is degraded *in vivo* to a natural metabolite (see Martin & Williams, Biochem. Eng. J. 16:97-105 (2003)).

The use of another polyhydroxyalkanoate, poly-3-hydroxybutyrate, formed into spheres of 5-100µm diameter, for embolization has been reported (see for example, Kassab, A. et al., J. Bioact. Compat. Polym. 14:291-303 (1999)). However, there are no reports of the use of poly-4-hydroxybutyrate in embolization. Notably, although poly-3-hydroxybutyrate and poly-4-hydroxybutyrate belong to the same class of materials, their polymer properties and chemical structures are substantially different. Poly-3-hydroxybutyrate is a rigid brittle material with a melting point around 170°C derived from a 3-hydroxyacid, whereas poly-4-hydroxybutyrate is derived from a 4-hydroxyacid, and is a strong, flexible and extensible material with a melting point around 60°C. Since it is highly crystalline, the degradation profile of poly-3-hydroxybutyrate is also much longer than that of poly-4-hydroxybutyrate (see Williams, S.F., et al. Applications of PHAs in Medicine and Pharmacy, in Biopolymers, Polyesters, III Vol. 4:91-127 (2002).

In one preferred embodiment, the particles have diameters ranging from 10 µm to 2,000µm, and are provided in the form of a dry powder or a suspension. The particles may be further sieved into more narrowly defined size ranges, for example, with distributions in sizes between the particles of 0-300 µm, and more preferably 0-200 µm. The size of a prophylactic or therapeutic dose will vary with the nature, type, location and severity of the condition to be treated and the route of administration. It will also vary with age, weight and the response of the patient. An effective amount of particles may range between a few dozen to a few hundred particles, but may be greater or smaller. One skilled in the art may chose to deliver particles of given size ranges, for example, a particle size range of 300-500µm, 500-700µm, or 700-900µm, could be selected for a specific procedure.

The exact size ranges required for each procedure can be readily determined by those skilled in the art.

In another preferred embodiment, the particles completely degrade after two weeks *in vivo,* more preferably after four weeks *in vivo,* and even more preferably after 12 weeks *in vivo.* In one embodiment, the particles comprise between about 0.5% to about 20% poly-4-hydroxybutrate and/or its copolymers by weight.

In yet another preferred embodiment, the particles can be suspended, do not agglomerate prior to use, and can be administered as an injectable suspension with a suitable liquid carrier.

In yet a further preferred embodiment, the particles have a shelf life greater than one year, and more preferably greater than three years. Additionally, a suspension of the particles may have a shelf life exceeding three months, more preferably six months, and even more preferably one year.

### Therapeutic, Prophylactic and Diagnostic Agents

In still yet another preferred embodiment, the particles may include a therapeutic, prophylactic or diagnostic or imaging agent. Examples include a dye, imaging agent, contrast agent, cell-adhesion factor, anti-angiogenic agent, and/or drug. Cell adhesion promoters include, but are not limited to, CM dextran, collagen, DEAE dextran, gelatin, glucosaminoglycans, fibronectin, lectins, polycations, and natural biological or synthetic cell adhesion agents. Examples of dyes that can be used to make direct visualization of the particles possible, include, but are not limited to, Cibacron Blue and Procion Red HE-3B. Examples of imaging agents, include, but are not limited to, magnetic resonance imaging agents such as erbium, gadolinium and magnetite. Examples of contrast agents that can be used include, but are not limited to, barium or iodine salts, iodipamide, and amino-3-triiodo-2, 4, 6-benzoic acid. Non-limiting examples of anti-angiogenic agents that may be incorporated are disclosed in U.S Patent No. 6,680,046 to Boschetti. Such components may be incorporated into the particles during their formation, or after their synthesis, for example by grafting or absorption.

### II. Methods to prepare Absorbable Embolization Particles

In a preferred embodiment, the absorbable embolization particles are prepared by an oil in water emulsion technique, as shown in examples 1-7.

In an alternative embodiment, the absorbable embolization particles are prepared by cutting poly-4-hydroxybutyrate filaments into defined lengths, as demonstrated by example 8.

In another alternative embodiment, the absorbable embolization particles may be prepared by extruding the spheres directly by underwater pelletization, or similar process.

The preferred method to sterilize the particles is exposure to ethylene oxide gas. Irradiation (gamma or electron beam) may also be used to sterilize the particles prior to injection into the patient.

### III. Methods of Administration of the Absorbable Embolization Particles

The absorbable embolization particles can be suspended, for example, in a physiologically acceptable liquid carrier, such as saline, aqueous solutions, or solutions containing sugars. Notably these liquid carriers may also contain cell adhesion promoters, marking agents, contrast agents, imaging agents, anti-angiogenic agents, or other drugs. The particles may be suspended just prior to use or supplied ready for use. Preferably the suspension is sterile.

Embolization is achieved by administering to a human or animal an injectable suspension comprising an effective amount of the particles, having diameters ranging from about 10µm to 2,000µm. The size of a prophylactic or therapeutic dose will vary with the nature, type, location and severity of the condition to be treated and the route of administration. It will also vary with age, weight and the response of the patient. An effective amount of particles may range between a few dozen to a few hundred particles, but may be greater or smaller. One skilled in the art may chose to deliver particles of given size ranges, for example, a particle size range of 300-500µm, 500-700µm, or 700-900µm, could be selected for a specific procedure.

Any suitable route may be used to administer the particles, including for example, parenteral, subcutaneous, or intramuscular, provided that it provides the patient with an effective dose at the desired target or location. The preferred route of administration is to the arteries via a catheter.

Conditions and disease states that can be prevented or treated by embolization include, but are not limited to, solid tumors, vascular malformations, and hemorrhagic events or processes. With respect to tumors; the embolization methods can be used to suppress pain, to limit blood loss occurring during surgical intervention following embolization, or to bring on tumor necrosis and to either avoid or minimize the necessity of surgical intervention. With respect to vascular malformations, the embolization methods can be used to normalize the blood flow to "normal" tissues, to aid in surgery and to limit the risk of hemorrhage. For hemorrhagic events or processes, the embolization methods can be used to reduce blood flow and to promote cicatrization of the arterial opening(s). In addition, the embolization methods can be used as a pre-surgical treatment in order to decrease the blood flow in blood rich organs (e.g., the liver) prior to surgical intervention. Examples of specific conditions that can be prevented or treated by the embolization methods include, but are not limited to, uterine tumors or fibroids; small intestinal hemorrhage, such as that associated with stress ulcer; surgical drain; anastomosis; tuberculous ulcer and nonspecific ulcer; symptomatic hepatic arteriovenous malformation (AVM); primary colorectal cancer; hepatocellular carcinomas; liver metastases; bone metastases; melanomas; cancers of the head or neck; and intracranial meningiomas.

### IV. Examples

### EXAMPLE 1: Poly-4-hydroxybutyrate (P4HB) microspheres prepared by an oil in water emulsion technique from dilute polymer solution.

Microspheres of P4HB were made using an oil in water emulsion technique. P4HB (8.4 g, lot # DC04-76-1, M_{w} 494,000 by GPC, Tepha, Inc., Cambridge, MA) was dissolved in methylene chloride (304 g, 230 ml) to prepare an 3.7% wt/vol solution. This polymer solution was added slowly with rapid overhead stirring to 2 L beaker containing an aqueous solution (0.5% wt/vol) of polyvinyl alcohol (89% hydrolyzed, M_{w} 31,000-50,000). Stirring was done using a 2-inch flat paddle at 820 RPM. The stirring was continued overnight and the methylene chloride was allowed to evaporate from the opened-top beaker. After complete evaporation of the methylene chloride, the stirring was stopped and the microsphere particles were allowed to settle. The supernatant was decanted and the microspheres were resuspended and washed in DI water three times.

The materials and conditions used in the following examples are provided in Table 1.

**Table 1. Experimental conditions for preparing poly-4-hydroxybutyrate (P4HB) microspheres.**

| **Example** | **4400** **g** | **CH₂Cl₂** **g** | **CH₂Cl₂** **Final vol.** **ml** | **Stirrer** **Speed** **(rpm)** | **Vol.** **0.5%** **PVA** | **Particle** **size** |
|---|---|---|---|---|---|---|
| 1 | 8.4 | 304 | 229* | 820 | 1500 | Small |
| 2 | 38.0 | 300 | 226* | 430 | 1500 | Large |
| 3 | 23.0 | 306 | 231* | 600 | 1500 | Table 2 |
| 4 | 34.5 | 459 | 346* | 595 | 2250 | Table 2 |
| 5 | 23.0 | 306 | 160 | 592 | 1500 | Table 2 |
| 6 | 23.1 | 305 | 185 | 594 | 1500 | Table 2 |
| 7 | 23.1 | 305 | 185 | 700 | 1500 | Table 2 |

| | | | | | | |
|---|---|---|---|---|---|---|
| * Some evaporation of methylene chloride may have occurred prior to mixing the polymer solution and PVA solution, resulting in a more concentrated solution of P4HB. | | | | | | |

### EXAMPLE 2: P4HB microspheres Prepared by an oil in water emulsion technique from a concentrated polymer solution

Microspheres of P4HB were made using an oil in water emulsion technique as in Example 1 except that a more concentrated solution of P4HB (38 g in 300 g, 226 ml methylene chloride) was used and stirring was done at lower speed (430 RPM) to produce larger P4HB microspheres.

### EXAMPLE 3: P4HB microspheres by an oil in water emulsion technique from a concentrated polymer solution.

Microspheres of P4HB were made using an oil in water emulsion technique as in Example 1 except that a more concentrated solution of P4HB (23 g in 306 g, 231 ml methylene chloride) was used and stirring was done at lower speed (600 RPM) to produce larger P4HB microspheres.

After washing and drying, 14.4 g of microspheres were collected (63% yield). Particles were sized by sieving and sizing data are shown in Table 2.

### EXAMPLE 4: P4HB microspheres prepared by an oil in water emulsion technique from a concentrated polymer solution.

Microspheres of P4HB were made using an oil in water emulsion technique as in Example 1 except that a more concentrated solution of P4HB (34.5 g in 459 g, 346 ml methylene chloride) was used and stirring was done at lower speed (595 RPM) to produce larger P4HB microspheres. Additionally, a greater volume (2250 ml) of PVA solution (0.5%) was used in a larger 4 L beaker.

After washing and drying of the microspheres, 125.9 g of microspheres were collected (75% yield). Particles were sized by sieving and sizing data are shown in Table 2.

### EXAMPLE 5: P4HB microspheres prepared by an oil in water emulsion technique from a concentrated polymer solution.

Microspheres of P4HB were made using an oil in water emulsion technique as in Example 1 except that a more concentrated solution of P4HB (23 g in 306 g, 231 ml methylene chloride) was used and stirring was done at lower speed (592 RPM) to produce larger P4HB microspheres.

After washing and drying, 19.0 g of microspheres were collected (83% yield). Particles were sized by sieving and sizing data are shown in Table 2.

### EXAMPLE 6: P4HB microspheres prepared by an oil in water emulsion technique from concentrated polymer solution.

Microspheres of P4HB were made using an oil in water emulsion technique as in Example 1 except that a more concentrated solution of P4HB (23.1 g in 305 g, 230 ml methylene chloride) was used and stirring was done at lower speed (594 RPM) to produce larger P4HB microspheres.

After washing and drying, 19.94 g of microspheres were collected (86% yield). Particles were sized by sieving and sizing data are shown in Table 2.

### EXAMPLE 7: P4HB microspheres prepared by an oil in water emulsion technique from concentrated polymer solution.

Microspheres of P4HB were made using an oil in water emulsion technique as in Example 1 except that a more concentrated solution of P4HB (23.1 g in 305 g, 230 ml methylene chloride) was used and stirring was done at lower speed (700 RPM) to produce larger P4HB microspheres.

After washing and drying, 18.79 g of microspheres were collected (81% yield). Particles were sized by sieving and sizing data are shown in Table 2.

### EXAMPLE 8: P4HB microspheres prepared from cut lengths of extruded P4HB fiber.

Melt extruded P4HB fiber 275 µm in diameter was cut into lengths of approximately 250 µm to create small particles of P4HB. These particles were less dense than a commercially available contrast agent (RenoCal 76, Bacco Diagnostics) and more dense than 0.9% saline solution but remained suspended in a 50:50 mixture of saline and contrast agent. The particles could be suspended in the solution of contrast and saline and delivered through a 4 F catheter.

### Table 2. Sizing data for microspheres produced by oil in water emulsion technique.

**Weight percent of particles sieved between selected sieves**

| Sample | > 500 µm | 500 - 355 µm | 355 -212 µm | < 212 µm |
|---|---|---|---|---|
| Example 3 | 1.9 | 11.7 | 58.7 | 27.7 |
| Example 4 | 0.22 | 0.29 | 3.0 | 96.5 |
| Example 5 | 64.1 | 15.2 | 14.3 | 6.5 |
| Example 6 | 65.1 | 19.9 | 11.0 | 4.0 |
| Example 7 | 18.6 | 35.0 | 33.3 | 13.1 |

## Claims

1. A composition for embolization in a human or animal, comprising particles of poly-4-hydroxybutyrate and/or its copolymers, wherein the particles have a diameter between 10µm and 2,000µm, degrade following implantation over a period of time of at least two weeks following implantation.

2. The composition of claim 1 wherein the particles are essentially uniform spheres.

3. The composition of claim 1 wherein the particles are between about 0.5% to about 20% poly-4-hydroxybutyrate by weight.

4. The composition of claim 1 wherein the particles have a size range of 300-500µm, 500-700µm, or 700-900µm.

5. The composition of claim 1 wherein the particles have a size range of 0-300 µm or 0-200 µm.

6. The composition of claim 1 wherein the particles do not aggregate.

7. The composition of claim 1 further comprising one or more agents selected from the group consisting of therapeutic, diagnostic and
prophylactic agents.

8. The composition of claim 7 wherein the agent is a diagnostic agent selected from the group consisting of contrast agents, dyes, and imaging agents.

9. The composition of claim 7 wherein the agent is a therapeutic selected from the group consisting of cell adhesion promoters, anti-angiogenic agents, and drugs.

10. The composition of claim 1 further comprising a pharmaceutically acceptable carrier for intravenous administration.

11. The composition of claim 11, wherein the particles do not clog during administration.

12. Use of a composition according to any one of Claims 1 to 11 in the manufacture of an injectable suspension for prophylactic or therapeutic embolization in a human or animal, in need of such embolization,

13. An injectable suspension of a composition according to any one of Claims 1 to 11 for use in prophylactic or therapeutic embolization in a human or animal in need of such embolization.

14. The use of Claim 12 or the injectable suspension of Claim 13 wherein the embolization is for prevention or treatment of a disorder selected from the group consisting of solid tumors, vascular malformations, and
hemorrhagic events or processes, including uterine tumors or fibroids; small intestinal hemorrhage, such as that associated with stress ulcer; surgical drain; anastomosis; tuberculous ulcer and nonspecific ulcer; symptomatic hepatic arteriovenous malformation (AVM); primary colorectal cancer; hepatocellular carcinomas; liver metastases; bone metastases; melanomas; cancers of the head or neck; and intracranial meningiomas.

15. The use of Claim 12 or the injectable suspension of Claim 13 wherein the embolization is for treatment of a tumor to suppress pain,
to limit blood loss occurring during surgical intervention following embolization, to bring on tumoral necrosis and to either avoid or minimize the necessity of surgical intervention.

16. The use of Claim 12 or the injectable suspension of Claim 13 wherein the embolization is for treatment of a vascular malformation to normalize the blood flow to normal tissues, to aid in surgery and to limit the risk of hemorrhage.

17. The use of Claim 12 or the injectable suspension of Claim 13 wherein the embolization is for treatment of hemorrhagic events or processes to reduce blood flow and to promote cicatrization of the arterial opening(s).

18. The use of Claim 12 or the injectable suspension of Claim 13 wherein the embolization is a pre-surgical treatment for decreasing the blood flow in blood rich organs (e.g., the liver) prior to surgical intervention.

## Patentansprüche

1. Zusammensetzung für die Embolisation in einem Menschen oder Tier, wobei die Zusammensetzung Partikel aus Poly-4-hydroxybutyrat und/oder seinen Copolymeren umfasst, wobei die Partikel einen Durchmesser zwischen 10 µm und 2000 µm haben und sich nach der Implantation innerhalb eines Zeitraums von wenigstens zwei Wochen nach der Implantation zersetzen.

2. Zusammensetzung nach Anspruch 1, wobei die Partikel im Wesentlichen einheitliche Kugeln sind.

3. Zusammensetzung nach Anspruch 1, wobei die Partikel zwischen ungefähr 0,5 Gew.-% und ungefähr 20 Gew.% Poly-4-hydroxybutyrat ausmachen.

4. Zusammensetzung nach Anspruch 1, wobei die Partikel einen Größenbereich von 300-500 µm, 500-700 µm oder 700-900 µm aufweisen.

5. Zusammensetzung nach Anspruch 1, wobei die Partikel einen Größenbereich von 0-300 µm oder 0-200 µm aufweisen.

6. Zusammensetzung nach Anspruch 1, wobei die Partikel nicht aggregieren.

7. Zusammensetzung nach Anspruch 1, die ferner ein oder mehrere Mittel umfasst, das bzw. die aus der Gruppe ausgewählt ist bzw. sind, die aus therapeutischen, diagnostischen und prophylaktischen Mitteln besteht.

8. Zusammensetzung nach Anspruch 7, wobei das Mittel ein diagnostisches Mittel ist, das aus der Gruppe ausgewählt ist, die aus Kontrastmitteln, Farbstoffen und Mitteln für eine bildgebende Darstellung besteht.

9. Zusammensetzung nach Anspruch 7, wobei das Mittel ein Therapeutikum ist, das aus der Gruppe ausgewählt ist, die aus die Zelladhäsion fördernden Mitteln, antiangiogenen Mitteln und Arzneimitteln besteht.

10. Zusammensetzung nach Anspruch 1, die ferner einen pharmazeutisch annehmbaren Träger für die intravenöse Verabreichung umfasst.

11. Zusammensetzung nach Anspruch 10, wobei sich die Partikel während der Verabreichung nicht zusammenballen.

12. Verwendung einer Zusammensetzung gemäß irgendeinem der Ansprüche 1 bis 11 bei der Herstellung einer injizierbaren Suspension für die prophylaktische oder therapeutische Embolisation bei einem Menschen oder Tier, der bzw. das diese Embolisation benötigt.

13. Injizierbare Suspension einer Zusammensetzung gemäß irgendeinem der Ansprüche 1 bis 11 für die Verwendung bei der prophylaktischen oder therapeutischen Embolisation bei einem Menschen oder Tier, der bzw. das diese Embolisation benötigt.

14. Verwendung nach Anspruch 12 oder injizierbare Suspension nach Anspruch 13, wobei die Embolisation der Prävention oder Behandlung einer Krankheit dient, die aus der Gruppe ausgewählt ist, die besteht aus soliden Tumoren, Gefäßmalformationen und hämorrhagischen Ereignissen oder Prozessen, einschließlich von Gebärmuttertumoren oder -fibroiden, kleinen Darmblutungen wie solchen, die mit Stressulzera assoziiert sind, chirurgischen Drainagen, Anastomosen, tuberkulösen Geschwüren und unspezifischen Geschwüren, symptomatischen hepatischen arteriovenösen Malformationen (AVM), primärem Kolorektalkrebs, hepatozellulären Karzinomen, Lebermetastasen, Knochenmetastasen, Melanomen, Krebsen des Kopfes oder Halses und intrakraniellen Meningiomen.

15. Verwendung nach Anspruch 12 oder injizierbare Suspension nach Anspruch 13, wobei die Embolisation der Behandlung eines Tumors zur Unterdrückung von Schmerzen, der Begrenzung des bei einem chirurgischen Eingriff nach der Embolisation auftretenden Blutverlustes, der Bewirkung einer Tumornekrose und entweder der Vermeidung oder der Minimierung der Notwendigkeit eines chirurgischen Eingriffs dient.

16. Verwendung nach Anspruch 12 oder injizierbare Suspension nach Anspruch 13, wobei die Embolisation der Behandlung einer Gefäßmalformation zur Normalisierung des Blutflusses zu normalen Geweben, der Unterstützung der chirurgischen Behandlung und der Begrenzung des Blutungsrisikos dient.

17. Verwendung nach Anspruch 12 oder injizierbare Suspension nach Anspruch 13, wobei die Embolisation der Behandlung hämorrhagischer Ereignisse oder Prozesse zur Verminderung des Blutflusses und zur Förderung der Vernarbung der Arterienöffnung(en) dient.

18. Verwendung nach Anspruch 12 oder injizierbare Suspension nach Anspruch 13, wobei die Embolisation eine voroperative Behandlung zur Erniedrigung des Blutflusses in blutreiche Organe (z. B. die Leber) vor einem chirurgischen Eingriff ist.

## Revendications

1. Composition pour l'embolisation chez un être humain ou un animal, comprenant des particules de poly-4-hydroxybutyrate et/ou ses copolymères, dans laquelle les particules ont un diamètre entre 10 µm et 2 000 µm, se dégradent après l'implantation sur une période de temps d'au moins deux semaines après l'implantation.

2. Composition selon la revendication 1, dans laquelle les particules sont des sphères essentiellement uniformes.

3. Composition selon la revendication 1, dans laquelle les particules ont environ 0,5 % environ 20 % de poly-4-hydroxybutyrate en poids.

4. Composition selon la revendication 1, dans laquelle les particules ont une gamme de tailles de 300 à 500 µm, 500 à 700 µm ou 700 à 900 µm.

5. Composition selon la revendication 1, dans laquelle les particules ont une gamme de tailles de 0 à 300 µm ou de 0 à 200 µm.

6. Composition selon la revendication 1, dans laquelle les particules ne s'agrègent pas.

7. Composition selon la a revendication 1, comprenant en outre un ou plusieurs agents choisis dans le groupe constitué par les agents thérapeutiques, diagnostiques et prophylactiques.

8. Composition selon la revendication 7, dans laquelle l'agent est un agent diagnostique choisi dans le groupe constitué par les agents de contraste, les colorants et les agents d'imagerie.

9. Composition selon la revendication 7, dans laquelle l'agent est un agent thérapeutique choisi dans le groupe constitué par les promoteurs d'adhesion cellulaire, les agents anti-angiogéniques et les substances médicamenteuses.

10. Composition selon la revendication 1, comprenant en outre un support pharmaceutiquement acceptable pour l'administration intraveineuse.

11. Composition selon la revendication 10, dans laquelle les particules ne forment pas un bouchon lors de l'administration.

12. Utilisation d'une composition selon l'une quelconque des revendications 1 à 11 dans la fabrication d'une suspension injectable pour l'embolisation prophylactique ou thérapeutique chez un être humain ou un animal ayant besoin d'une telle embolisation.

13. Suspension injectable d'une composition selon l'une quelconque des revendications 1 à 11, pour l'utilisation dans l'embolisation prophylactique ou thérapeutique chez un être humain ou un animal ayant besoin d'une telle embolisation.

14. Utilisation selon la revendication 12 ou suspension injectable selon la revendication 13, dans laquelle l'embolisation est destinée à prévenir ou traiter un trouble choisi dans le groupe constitué par les tumeurs solides, les malformations vasculaires et les événements ou processus hémorragiques, incluant les tumeurs ou fibromes utérins ; une hémorragie de l'intestin grêle, comme celle associée à un ulcère de stress ; un drain chirurgical ; une anastomose ; un ulcère tuberculeux et un ulcère non spécifique ; une malformation artério-veineuse (MAV) hépatique symptomatique ; un cancer colorectal primaire ; les carcinomes hépatocellulaires ; les métastases hépatiques ; les métastases osseuses ; les mélanomes ; les cancers de la tête ou du cou ; et les méningiomes intracrâniens.

15. Utilisation selon la revendication 12 ou suspension injectable selon la revendication 13, dans laquelle l'embolisation est destinée au traitement d'une tumeur pour supprimer une douleur, pour limiter une perte de sang se produisant pendant une intervention chirurgicale après une embolisation, pour provoquer une nécrose tumorale et pour soit éviter soit minimiser la nécessité d'une intervention chirurgicale.

16. Utilisation selon la revendication 12 ou suspension injectable selon la revendication 13, dans laquelle l'embolisation est destinée au traitement d'une malformation vasculaire pour normaliser le flux sanguin aux tissus normaux, pour faciliter une opération chirurgicale et pour limiter le risque d'hémorragie.

17. Utilisation selon la revendication 12 ou suspension injectable selon la revendication 13, dans laquelle l'embolisation est destinée au traitement d'événements ou processus hémorragiques pour réduire le flux sanguin et pour activer la cicatrisation de la (des) ouverture(s) artérielle(s).

18. Utilisation selon la revendication 12 ou suspension injectable selon la revendication 13, dans laquelle l'embolisation est un traitement préchirurgical pour diminuer le flux sanguin dans les organes riches en sang (par exemple, le foie) avant une intervention chirurgicale.
